# FASCICULE DE BREVET EUROPEEN

(11) **EP 2 821 386 B1**
(45) Date de publication et mention de la délivrance du brevet: **17.08.2022**
(21) Numéro de dépôt: 14180362.7
(22) Date de dépôt: 16.09.2010
(51) Int. Cl.: A01N 31/16, A01P 1/00, A01P 3/00, C07C 49/245, A61K 8/35, A61K 47/10, A61Q 19/00, C07C 49/255

(54) **Composition cosmétique, dermatologique ou pharmaceutique comprenant au moins un dérivé de vanilline comme conservateur**
Kosmetische, dermatologische oder pharmazeutische Zusammensetzung enthaltend wenigstens ein Vanillinderivate als Konservierungsmittel
Cosmetic, dermatological or pharmaceutical compositions comprising at least one vanillin derivative as a preservative

(30) Priorité: 01.10.2009 FR 0956843; 06.10.2009 US 248999 P; 17.11.2009 FR 0958082
(43) Date de publication de la demande: 07.01.2015
(62) Demande divisionnaire de: 10770573.3
(73) Titulaire: L'OREAL, 75008 Paris (FR)
(72) Inventeur: Dalko, Maria, 78000 Versailles (FR)
(74) Mandataire: L'Oreal

(56) Documents cités:
- WO-A1-2008/071027
- WO-A2-2009/019255
- JP-A- 5 168 401
- US-A1- 2005 196 492
- US-A1- 2008 253 976
- WINTER ET AL: "Odeur et constitution XIX. Sur des homologues et analogues de la p-hydroxyphényl-1-butanone-3 ( cétone de framboise ) // Odor and constitution. XIX. Homologs and analogs of 1-(p-hydroxyphenyl)-3-but anone (raspberry ketone)", 1 janvier 1961 (1961-01-01), HELVETICA CHIMICA ACTA, VERLAG HELVETICA CHIMICA ACTA, BASEL, CH LNKD- DOI:10.1002/HLCA.19610440738, PAGE(S) 2110 - 2121, XP009133368, ISSN: 0018-019X [extrait le 2004-10-24] * tableau I; composé XVIII *

## Description

La présente invention concerne une composition cosmétique, dermatologique ou pharmaceutique, comprenant de dérivés de vanilline notamment comme conservateur

Il est courant d'introduire dans les compositions cosmétiques ou dermatologiques, des conservateurs chimiques destinés à lutter contre le développement des microorganismes dans ces compositions, ce qui les rendrait rapidement inaptes à être utilisées. Il faut notamment protéger les compositions contre les microorganismes susceptibles de se développer à l'intérieur de la composition, par exemple lors de leur fabrication, et également contre ceux que l'utilisateur pourrait y introduire en la manipulant, en particulier lors de la préhension avec les doigts de produits en pot. Des conservateurs chimiques couramment utilisés sont notamment les parabènes, les acides organiques ou les composés libérateurs de formol. Ces conservateurs présentent toutefois l'inconvénient de causer des irritations, en particulier sur les peaux sensibles, lorsqu'ils sont présents à des taux relativement importants. Par ailleurs, dans le souci de l'environnement, les consommateurs sont de plus en plus en recherche d'agents de conservation respectueux de l'environnement, notamment non écotoxiques. Le document WO 2008/07027 décrit des compositions cosmétiques de soin comprenant des dérivés de benzaldéhydes dont la vaniline, utilisées comme agents antibactériens à large spectre. De telles compositions avec la vaniline peuvent poser un problème de stabilité au stokage. L'efficacité des conservateurs utilisés classiquement est variable et leur formulation peut donc poser des problèmes, notamment d'incompatibilité, voire de déstabilisation, des formules, en particulier des émulsions.

La présente invention a pour but de proposer de nouveaux agents conservateurs présentant notamment un spectre antimicrobien large, au moins aussi large, voire supérieur, à celui des composés déjà existants, et ne présentant pas les inconvénients de l'art antérieur, en particulier ayant des propriétés physicochimiques spécifiques permettant de protéger les formules cosmétiques de la contamination microbienne tout en étant bien tolérés.

L'objet de l'invention est donc une composition cosmétique, dermatologique ou pharmaceutique comprenant au moins un composé de formule (I) : 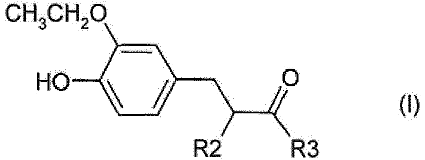 dans laquelle :
- soit R2 représente un atome d'hydrogène, et R3 représente un radical alkyle (saturé) linéaire en C1-C6, éventuellement substitué par un groupe hydroxyle; ou bien un radical alcényle (insaturé C=C) linéaire en C2-C6, ou encore un radical alcényle linéaire en C2-C12 substitué par un groupe hydroxyle;
- soit R2 représente un radical méthyle ou éthyle, et R3 représente un radical alkyle (saturé) linéaire en C1-C12, éventuellement substitué par un groupe hydroxyle; ou bien un radical alcényle (insaturé C=C) linéaire en C2-C12, éventuellement substitué par un groupe hydroxyle ;
la composition se présentant sous la forme de solutions hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules choisies parmi les nanoparticules polymérigues telles que les nanosphères et les nanocapsules, ou les vésicules lipidiques de type ionique et/ou non ionique (liposomes, niosomes, oléosomes), de nanoémulsions et
se présentant sous la forme d'un produit de maquillage de la peau du visage, du corps ou des lèvres; d'un gel ou lotion après-rasage; d'une crème dépilatoire; d'une composition d'hygiène corporelle telle qu'un gel douche ou un shampooing; d'une composition pharmaceutique; d'une composition solide telle qu'un savon ou un pain de nettoyage, d'une composition pour aérosol comprenant également un agent propulseur sous pression; d'une lotion de mise en plis, d'une crème ou d'un gel coiffant, d'une composition de teinture, d'une lotion restructurante pour cheveux, d'une composition de permanente, d'une lotion ou d'un gel antichute.

Par agent conservateur, on entend une substance qui est ajoutée communément à une composition afin d'en assurer sa conservation vis-à-vis d'un agent contaminant. Avantageusement, les composés de formule (I) selon l'invention sont utilisés comme agent antimicrobien et/ou antibactérien et/ou antifongique.

De préférence, les composés répondent à la formule (I), dans laquelle :
- (i) R2 est H et R3 représente un radical méthyle, éthyle, propyle, butyle ou pentyle, éventuellement substitué par un OH et notamment de structure -CH₂-CH(OH)-R5 avec R5 représentant un radical alkyle linéaire en C1-C4; ou un radical alcényle en C2-C6, notamment un radical -CH=CH-R4 avec R4 représentant un radical alkyle linéaire en C1-C4; ou bien
- (ii) R2 représente CH₃ et R3 représente (i) un radical alkyle en C1-C10, notamment méthyle, éthyle, propyle, butyle, pentyle ou hexyle; (ii) un radical alcényle en C2-C10, notamment un radical -CH=CH-R4 avec R4 représentant un radical alkyle linéaire en C1-C6; ou encore (iii) un radical hydroxyalkyle de structure -CH₂-CH(OH)-R5 avec R5 représentant un radical alkyle linéaire en C1-C10, de préférence en C4-C10.

On peut bien évidemment utiliser un mélange de composés de formule (I).

De préférence, la composition ne comprend pas d'autres agents conservateurs que ceux de formule (I). En particulier, la composition ne contient pas de parabens.

Particulièrement les composés de formule (I) sont de formule (I') ci-après : 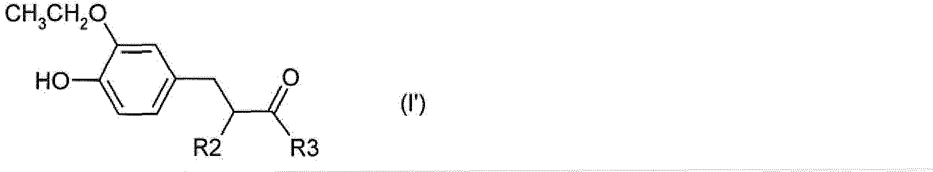 dans laquelle :
- soit R2 représente un atome d'hydrogène, et R3 représente un radical alkyle (saturé) linéaire en C2-C6, éventuellement substitué par un groupe hydroxyle; ou bien un radical alcényle (insaturé C=C) linéaire en C2-C6, ou encore un radical alcényle linéaire en C2-C12 substitué par un groupe hydroxyle;
- soit R2 représente un radical méthyle ou éthyle, et R3 représente un radical alkyle (saturé) linéaire en C1-C12, éventuellement substitué par un groupe hydroxyle; ou bien un radical alcényle (insaturé C=C) linéaire en C2-C12, éventuellement substitué par un groupe hydroxyle.

De préférence, dans la formule (I'),
- (i) R2 est H et R3 représente un radical éthyle, propyle, butyle ou pentyle, éventuellement substitué par un OH et notamment de structure -CH₂-CH(OH)-R5 avec R5 représentant un radical alkyle linéaire en C1-C4; ou un radical alcényle en C2-C6, notamment un radical -CH=CH-R4 avec R4 représentant un radical alkyle linéaire en C1-C4; ou
- (ii) R2 représente CH₃ et R3 représente (i) un radical alkyle en C1-C10, notamment méthyle, éthyle, propyle, butyle, pentyle ou hexyle; (ii) un radical alcényle en C2-C10, notamment un radical -CH=CH-R4 avec R4 représentant un radical alkyle linéaire en C1-C6; ou encore (iii) un radical hydroxyalkyle de structure -CH₂-CH(OH)-R5 avec R5 représentant un radical alkyle linéaire en C1-C10, de préférence en C4-C10.

On peut particulièrement citer les composés de formule (I) ou (I') suivants : 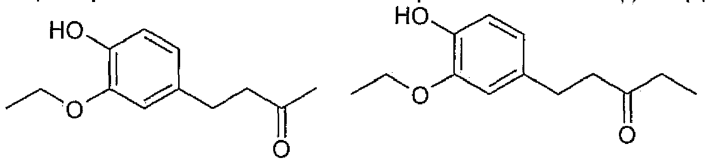 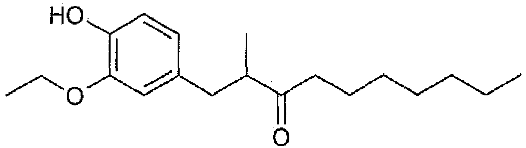 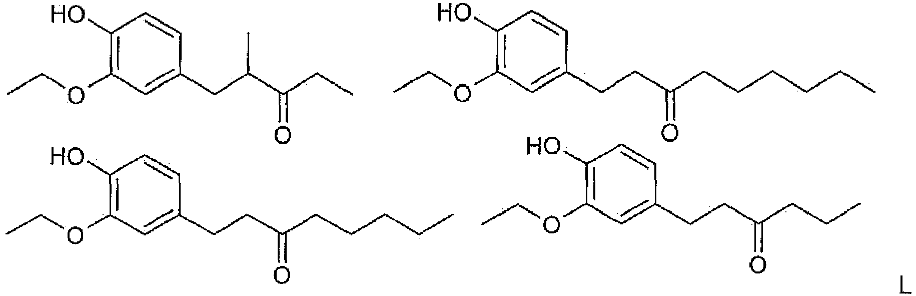 es compositions cosmétiques, dermatologiques ou pharmaceutiques comprenant au moins un composé de formule (I), ou de formule (I'), forment également un objet de la présente invention.

Les composés de formule (I) peuvent être préparés aisément par l'homme du métier sur la base de ses connaissances générales. On peut notamment citer les références bibliographiques suivantes : J. Asian Natural Products Research, 2006, 8(8), 683-688; Helv. Chimica Acta, 2006, 89(3), 483-495; Chem. Pharm. Bull., 2006, 54(3), 377-379; et Bioorg. Med. Chem. Lett., 2004, 14(5), 1287-1289.

Ils peuvent ainsi être préparés à partir d'éthylvanilline de la manière suivante :

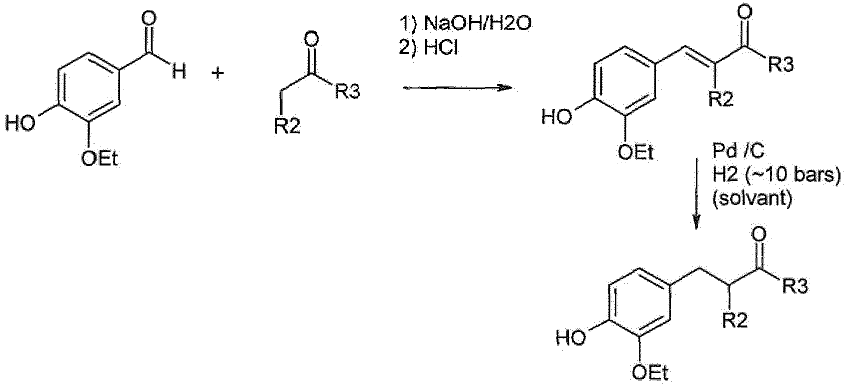

Les composés de formule (I), seul ou en mélange, peuvent être employés à raison de 0,01 à 5% en poids, notamment 0,1 à 2,5% en poids, par rapport au poids de la composition, dans les compositions cosmétiques, dermatologiques ou pharmaceutiques.

Les compositions cosmétiques, dermatologiques ou pharmaceutiques comprennent par ailleurs un milieu cosmétiquement, dermatologiquement ou pharmaceutiquement acceptable, c'est-à-dire compatible avec les matières kératiniques telles que la peau du visage ou du corps, les lèvres, les cheveux, les cils, les sourcils et les ongles.

Les compositions selon l'invention peuvent se présenter sous toutes les formes galéniques classiquement utilisées, notamment pour une application topique, et sous forme de solutions hydroalcooliques, d'émulsions huile-dans-eau (H/E) ou eau-dans-huile (E/H) ou multiple (triple : E/H/E ou H/E/H), de gels aqueux, ou de dispersions d'une phase grasse dans une phase aqueuse à l'aide de sphérules choisies parmi es nanoparticules polymériques telles que les nanosphères et les nanocapsules, ou des vésicules lipidiques de type ionique et/ou non ionique (liposomes, niosomes, oléosomes), de nanoémulsions. Ces compositions sont préparées selon les méthodes usuelles.

Les compositions selon l'invention peuvent être plus ou moins fluides et avoir l'aspect d'une crème blanche ou colorée, d'une pommade, d'un lait, d'une lotion, d'un sérum, d'une pâte, d'une mousse. Elles peuvent être éventuellement appliquées sur la peau sous forme d'aérosol. Elles peuvent aussi se présenter sous forme solide, et par exemple sous forme de stick.

La composition selon l'invention se présentef sous la forme :
- d'un produit de maquillage de la peau du visage, du corps ou des lèvres;
- d'un gel ou lotion après-rasage;
- d'une crème dépilatoire;
- sous la forme d'une composition d'hygiène corporelle telle qu'un gel douche ou un shampooing;
- d'une composition pharmaceutique;
- d'une composition solide telle qu'un savon ou un pain de nettoyage;
- d'une composition pour aérosol comprenant également un agent propulseur sous pression.; ou
- d'une lotion de mise en plis, d'une crème ou d'un gel coiffant, d'une composition de teinture, d'une lotion restructurante pour cheveux, d'une composition de permanente, d'une lotion ou d'un gel antichute;

Le milieu physiologiquement acceptable dans lequel les composés peuvent être employés, ainsi que ses constituants, leur quantité, la forme galénique de la composition et son mode de préparation, peuvent être choisis par l'homme du métier sur la base de ses connaissances générales en fonction du type de composition recherchée.

Notamment, la composition peut comprendre tout corps gras usuellement utilisé dans le domaine d'application envisagé. On peut notamment citer les corps gras siliconés tels que les huiles, les gommes et les cires de silicone, ainsi que les corps gras non siliconés tels que les huiles, les pâteux et les cires d'origine végétale, minérale, animale et/ou synthétique. Les huiles peuvent éventuellement être volatiles ou non volatiles.

Parmi les huiles de silicone, on peut citer les polydiméthylsiloxanes (PDMS) volatiles ou non à chaîne siliconée linéaire ou cyclique, liquides ou pâteux à température ambiante, notamment les cyclopolydiméthylsiloxanes telles que la cyclohexasiloxane; les polydiméthylsiloxanes comportant des groupements alkyle, alcoxy ou phényle, pendant ou en bout de chaîne siliconée, groupements ayant de 2 à 24 atomes de carbone; les silicones phénylées comme les phényltriméthicones, les phényldiméthicones, les phényltriméthylsiloxydiphényl-siloxanes, les diphényl-diméthicones, les diphénylméthyldiphényl trisiloxanes, les 2-phényléthyltriméthyl-siloxysilicates, et les polyméthylphénylsiloxanes;

Parmi les huiles hydrocarbonées d'origine végétale, on peut citer les triglycérides liquides d'acides gras comportant de 4 à 10 atomes de carbone comme les triglycérides des acides heptanoïque ou octanoïque ou encore, par exemple les huiles de tournesol, de maïs, de soja, de courge, de pépins de raisin, de sésame, de noisette, d'abricot, de macadamia, d'arara, de tournesol, de ricin, d'avocat, les triglycérides des acides caprylique/caprique; l'huile de jojoba, l'huile de beurre de karité;

On peut encore citer comme corps gras susceptible d'être employé :
- les acides gras ayant de 8 à 32 atomes de carbone;
- les esters et les éthers de synthèse, notamment de formule R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone, comme par exemple l'huile de Purcellin, l'isononanoate d'isononyle, le myristate d'isopropyle, le palmitate d'éthyl-2-hexyle, le stéarate d'octyl-2-dodécyle, l'érucate d'octyl-2-dodécyle, l'isostéarate d'isostéaryle; les esters hydroxylés comme l'isostéaryllactate, l'octylhydroxystéarate, l'hydroxystéarate d'octyldodécyle, le diisostéarylmalate, le citrate de triisocétyle, les heptanoates, octanoates, décanoates d'alcools gras; les esters de polyol, comme le dioctanoate de propylène glycol, le diheptanoate de néopen-tylglycol et le diisononanoate de diéthylèneglycol; et les esters du pentaérythritol comme le tétraisostéarate de pentaérythrityle;

- les hydrocarbures linéaires ou ramifiés, d'origine minérale ou synthétique, tels que les huiles de paraffine, volatiles ou non, et leurs dérivés, la vaseline, les poly-décènes, le polyisobutène hydrogéné tel que l'huile de parléam;
- les alcools gras ayant de 8 à 26 atomes de carbone, comme l'alcool cétylique, l'alcool stéarylique et leur mélange (alcool cétylstéarylique), l'octyldodécanol, le 2-butyloctanol, le 2-hexyldécanol, le 2-undécylpentadécanol, l'alcool oléique ou l'alcool linoléique.

La composition peut également comprendre un milieu aqueux qui comprend de l'eau, un milieu hydroalcoolique contenant un alcool en C2-C6 tel que l'éthanol ou l'isopropanol, ou un milieu organique comprenant des solvants organiques usuels tels que des alcools en C2-C6, notamment l'éthanol et l'isopropanol, des glycols tels que le propylène glycol, des cétones.

La composition selon l'invention peut également comprendre les adjuvants habituels dans les domaines cosmétique et dermatologique, tels que les épaississants, les ëmulsionnants, les tensioactifs, les gélifiants, les actifs cosmétiques, les parfums, les charges, les matières colorantes, les hydratants, les vitamines, les polymères. Les quantités de ces différents adjuvants sont celles classiquement utilisées dans les domaines considérés, par exemple de 0,001 à 20 % du poids total de la composition. Ces adjuvants, ainsi que leurs concentrations, doivent être tels qu'ils ne nuisent pas aux propriétés avantageuses des composés selon l'invention.

Le pH des compositions selon l'invention, lorsqu'elles comprennent au moins une phase aqueuse (solutions aqueuses, émulsions par exemple), est de préférence compris entre 4 et 9, de préférence entre 4 et 7, avantageusement entre 5 et 6.

L'invention est illustrée plus en détail dans les exemples de réalisation suivants.

### Exemple 1 : détermination de l'activité antimicrobienne d'un composé selon l'invention

L'efficacité antimicrobienne d'un composé de formule (I) a été évaluée par la méthode du Challenge Test ou contamination artificielle.

### Composé testé:

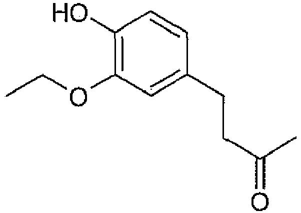

### Protocole

La méthode du challenge-test consiste en une contamination artificielle de l'échantillon par des souches microbiennes de collection (bactéries, levures et moisissures) et en une évaluation du nombre de micro-organismes revivifiables sept jours après l'inoculation.

Afin de mettre en évidence l'effet de composés de formule (I), l'activité antimicrobienne d'une formule cosmétique contenant respectivement 2% de composé selon l'invention a été comparée avec la même formule seule (témoin), après inoculation d'environ 10⁶ UFC (Unités formant des colonies)/gramme de formule cosmétique.

### Formule cosmétique (% en poids)

| | |
|---|---|
| - Tristéarate de sorbitane (Span 65 V^{®} de Croda) | 0,9% |
| - Stéarate de polyéthylène glycol (40 OE) (Myrj 52 P^{®} de Croda) | 2,0% |
| - Mélange de mono-distéarate (36/64) de glycéryle/ stéarate de potassium | 3,0% |
| - Acides gras d'origine végétale (acide stéarique / acide palmitique/ acide myristique 53/44/3) | 1,0% |
| - Alcool cétylique | 3,8% |
| - Myristyl myristate | 2,0% |
| - Cyclopentasiloxane | 5,0% |
| - Charges | 0,8% |
| - Glycérine | 3,0% |
| - Isoparaffine hydrogénée | 7,2% |
| - Vaseline blanche | 4,0% |
| - Eau | qsp 100% |

### Cultures de microorganismes

5 cultures pures de microorganismes sont utilisées.

| GERMES | Milieu de repiquage | T° | ATCC |
|---|---|---|---|
| *Escherichia coli* (Ec) | Trypto-caséine soja | 35°C | 8739 |
| *Enterococcus faecalis* (Ef) | Trypto-caséine soja | 35°C | 33186 |
| *Pseudomonas aeruginosa* (Pa) | Trypto-caséine soja | 35°C | 19429 |
| *Candida albicans* (Ca) | Sabouraud | 35°C | 10231 |
| *Aspergillus niger* (An) | Malt | 35°C | 6275 |

| | | | |
|---|---|---|---|
| ATCC = American Type Culture Collection | | | |

Les souches de bactéries gram - (*Escherichia coli* et *Pseudomonas aeruginosa),* bactérie gram + (*Enterococcus faecalis),* levure *(Candida albicans*), et moisissure *(Aspergillus niger)* sont ensemencées dans du milieu de repiquage, respectivement la veille de l'inoculation pour les bactéries et la levure, et 5 jours avant l'inoculation pour la moisissure.

Le jour de l'inoculation :
- on prépare respectivement pour les bactéries et la levure, une suspension dans du diluant Tryptone sel, de manière à obtenir au spectrophotomètre une suspension de densité optique comprise entre 35% et 45% de lumière transmise à 544 nm;
- pour la moisissure, on prélève les spores en lavant l'agar avec 6 à 7 ml de solution de récolte et on récupère la suspension dans un flacon ou un tube stérile.

Après avoir homogénéisé la suspension microbienne, on introduit dans chaque pilulier 0,2 ml d'inoculum (les suspensions sont utilisées pures: entre 1×10⁸ et 3×10⁸ UFC par ml) et on homogénéise parfaitement à l'aide d'une spatule la suspension microbienne dans les 20 g de produit (= formule cosmétique).

Le taux de micro-organismes présents dans le produit correspond après homogénéisation à une concentration de 10⁶ germes par gramme de produit soit l'inoculation à 1% d'un inoculum à 10⁸ germes par ml.

Après 7 jours de temps de contact entre les germes et le produit à 22°C ± 2°C et à l'obscurité, on réalise des dilutions décimales et on dénombre le nombre de micro-organismes revivifiables restant dans le produit.

### Résultats

| | | Nb d'UFC/ gramme de produit à T7 jours | | | | |
|---|---|---|---|---|---|---|
| | Taux | *E.coli* | *P. aeruginosa* | *E. faecalis* | *C. albicans* | *A. niger* |
| Composé | 2% | <200 | <200 | <200 | <200 | 3,4.10⁵ |

| | | | | | | |
|---|---|---|---|---|---|---|
| <200 UFC : seuil de sensibilité de la méthode | | | | | | |

### Exemple 2

| On prépare une émulsion comprenant (% en poids): | |
|---|---|
| - Tristéarate de sorbitane (Span 65 V^{®} de Croda) | 0,9% |
| - Stéarate de polyéthylène glycol (40 OE) (Myrj 52 P^{®} de Croda) | 2% |
| - Mélange de mono-distéarate (36/64) de glycéryle/ stéarate de potassium | 3% |
| - Acides gras d'origine végétale (acide stéarique / acide palmitique/ acide myristique 53/44/3) | 1% |
| - Glycérine | 3% |
| - Cyclopentasiloxane | 5% |
| - Isoparaffine hydrogénée | 7,2% |
| - Vaseline blanche | 4% |
| - Alcool cétylique | 4% |
| - Myristyl myristate | 2% |
| - Charges | 0,8% |
| - composé testé dans l'exemple 1 | 2% |
| - Eau | qsp 100% |

### Exemple 3

| On prépare une émulsion H/E comprenant (% en poids) : | |
|---|---|
| - hydroxyde de sodium | 0,03% |
| - huile de vaseline | 10% |
| - palmitate de 2-éthyle hexyle | 10% |
| - copolymère acide acrylique/méthacrylate de stéaryle polymérisé dans un mélange acétate d'éthyle/cyclohexane | 0,1% |
| - glycérol | 5% |
| - mélange cétylstearyl glucoside et d'alcools cétylique, stéarylique (12/46/42) | 2,45% |
| - composé testé dans l'exemple 1 | 2% |
| - eau désionisée microbiologiquement propre | qsp 100% |

### Exemple 4

| On prépare une lotion comprenant (% en poids) : | |
|---|---|
| - allantoïne | 0,05% |
| - chlorure de sodium | 0,09% |
| - acide citrique | qsp pH 7 ± 0,2 |
| - eau de bleuet | 1% |
| - hexylène glycol (2 methyl-2,4 pentanediol) | 1% |
| - glycérol | 5% |
| - N-cocoylamidoethyl, N-ethoxycarboxymethyl glycinate de sodium | 1,1% |
| - lauryl éther sulfate de sodium / magnésium (80/20) 4OE (52% MS) | 0,45% |
| - composé testé dans l'exemple 1 | 1,5% |
| - eau désionisée microbiologiquement propre | qsp 100% |

## Revendications

1. Composition cosmétique, dermatologique ou pharmaceutique, comprenant au moins un composé de formule (I) : 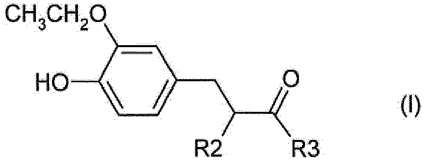 dans laquelle :
- soit R2 représente un atome d'hydrogène, et R3 représente un radical alkyle (saturé) linéaire en C1-C6, éventuellement substitué par un groupe hydroxyle; ou bien un radical alcényle (insaturé C=C) linéaire en C2-C6, ou encore un radical alcényle linéaire en C2-C12 substitué par un groupe hydroxyle;
- soit R2 représente un radical méthyle ou éthyle, et R3 représente un radical alkyle (saturé) linéaire en C1-C12, éventuellement substitué par un groupe hydroxyle; ou bien un radical alcényle (insaturé C=C) linéaire en C2-C12, éventuellement substitué par un groupe hydroxyle ;
la composition se présentant sous la forme de solutions hydroalcooliques ; d'émulsions huile-dans-eau ou eau-dans-huile ou multiple, de gels aqueux, ou de dispersions d'une phase grasse dans une phase aqueuse à l'aide de sphérules choisies parmi les nanoparticules ou des vésicules lipidiques de type ionique et/ou non ionique
et
se présentant sous la forme d'un produit de maquillage de la peau du visage, du corps ou des lèvres; d'un gel ou lotion après-rasage; d'une crème dépilatoire; d'une composition d'hygiène corporelle telle qu'un gel douche ou un shampooing; d'une composition pharmaceutique; d'une composition solide telle qu'un savon ou un pain de nettoyage; d'une composition pour aérosol comprenant également un agent propulseur sous pression; d'une lotion de mise en plis, d'une crème ou d'un gel coiffant, d'une composition de teinture, d'une lotion restructurante pour cheveux, d'une composition de permanente, d'une lotion ou d'un gel antichute.

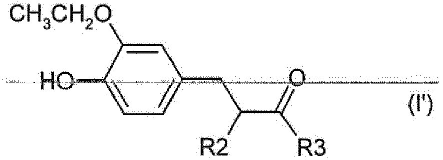

2. Composition selon la revendications précédentes, dans laquelle le composé de formule (I) est choisi parmi les composés suivants:

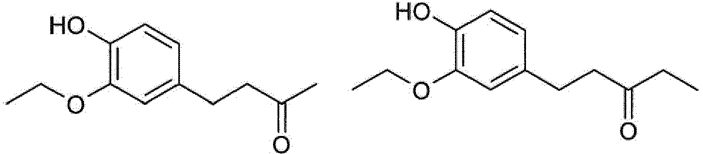

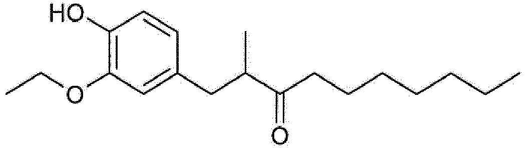

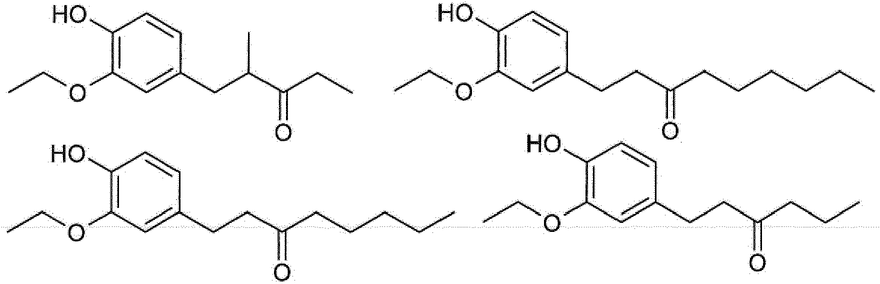

3. Composition selon l'une des revendications précédentes, dans laquelle le composé de formule (I) est choisi parmi les composés suivants:

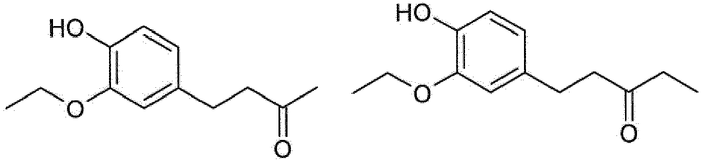

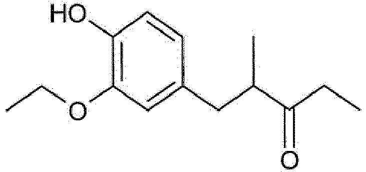

4. Composition selon l'une des revendications précédentes, dans laquelle le composé de formule (I) est le composé :

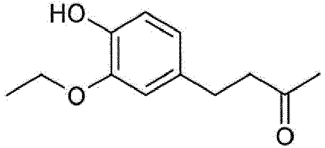

5. Composition selon l'une des revendications précédentes, dans laquelle le composé de formule (I), seul ou en mélange, est présent à raison de 0,01 à 5% en poids, notamment 0,1 à 2,5% en poids, par rapport au poids de la composition.

6. Composition selon l'une des revendications précédentes, se présentant sous la forme d'une crème blanche ou colorée, d'une pommade, d'un lait, d'une lotion, d'un sérum, d'une pâte, d'une mousse, de stick.

7. Composition selon l'une des revendications précédentes, comprenant un milieu physiologiquement acceptable qui comprend au moins un ingrédient choisi parmi les corps gras siliconés tels que les huiles, les gommes et les cires de silicone; les corps gras non siliconés tels que les huiles, les pâteux et les cires d'origine végétale, minérale, animale et/ou synthétique; les acides gras ayant de 8 à 32 atomes de carbone; les esters et les éthers de synthèse, notamment de formule R¹COOR² et R¹OR² dans laquelle R¹ représente le reste d'un acide gras comportant de 8 à 29 atomes de carbone, et R² représente une chaîne hydrocarbonée, ramifiée ou non, contenant de 3 à 30 atomes de carbone; les hydrocarbures linéaires ou ramifiés, d'origine minérale ou synthétique; les alcools gras ayant de 8 à 26 atomes de carbone; de l'eau; les alcools en C2-C6; les glycols tels que le propylène glycol, les cétones; les épaississants, les émulsionnants, les tensioactifs, les gélifiants, les actifs cosmétiques, les parfums, les charges, les matières colorantes, les hydratants, les vitamines, les polymères.

8. Composition selon l'une des revendications précédentes, se présentant sous la forme d'un produit de maquillage de la peau du visage, du corps ou des lèvres.

9. Composition selon l'une des revendications précédentes, se présentant sous la forme d'un shampooing.

10. Composition selon l'une des revendications précédentes, se présentant sous la forme d'une crème ou d'un gel coiffant.

11. Composition selon l'une des revendications précédentes, se présentant sous la forme d'une composition de teinture.

12. Composition selon l'une des revendications précédentes, se présentant sous la forme d'une lotion ou d'un gel antichute.

## Patentansprüche

1. Kosmetische, dermatologische oder pharmazeutische Zusammensetzung, umfassend mindestens eine Verbindung der Formel (I): 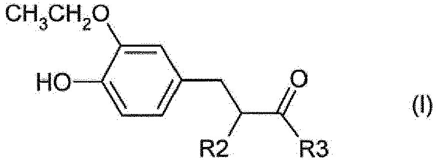 worin:
- entweder R2 für ein Wasserstoffatom steht und R3 für einen (gesättigten) linearen C1-C6-Alkylrest, der gegebenenfalls durch eine Hydroxylgruppe substituiert ist; oder auch einen (C=C-ungesättigten) linearen C2-C6-Alkenylrest oder auch einen linearen C2-C12-Alkenylrest, der durch eine Hydroxylgruppe substituiert ist, steht;
- oder R2 für einen Methyl- oder Ethylrest steht und R3 für einen (gesättigten) linearen C1-C12-Alkylrest, der gegebenenfalls durch eine Hydroxylgruppe substituiert ist; oder auch einen (C=C-ungesättigten) linearen C2-C12-Alkenylrest, der gegebenenfalls durch eine Hydroxylgruppe substituiert ist, steht;
wobei die Zusammensetzung in Form von wässrigalkoholischen Lösungen, Öl-in-Wasser- oder Wasserin-Öl- oder Mehrfachemulsionen, wässrigen Gelen oder Dispersionen einer Fettphase in einer wässrigen Phase mit Hilfe von Kügelchen, die aus Nanopartikeln oder Lipidvesikeln vom ionischen oder nichtionischen Typ ausgewählt sind, vorliegt
und
in Form eines Produkts zum Schminken der Haut des Gesichts, des Körpers oder der Lippen; eines Aftershave-Gels oder einer Aftershave-Lotion; einer Enthaarungscreme; einer Körperhygienezusammensetzung wie eines Duschgels oder eines Shampoos; einer pharmazeutischen Zusammensetzung; einer festen Zusammensetzung wie einer Seife oder eines Reinigungsstücks; einer Aerosolzusammensetzung, die auch ein unter Druck stehendes Treibmittel umfasst; einer Haarfestigungslotion, einer Frisiercreme oder eines Frisiergels, einer Färbezusammensetzung, einer Lotion zur Restrukturierung der Haare, einer Dauerwellenzusammensetzung, einer Lotion oder eines Gels gegen Haarausfall vorliegt.

2. Zusammensetzung nach dem vorhergehenden Anspruch, wobei die Verbindung der Formel (I) aus den folgenden Verbindungen ausgewählt ist:

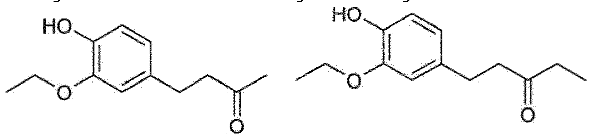

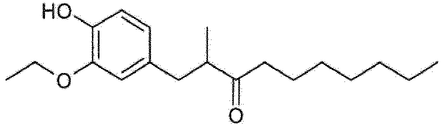

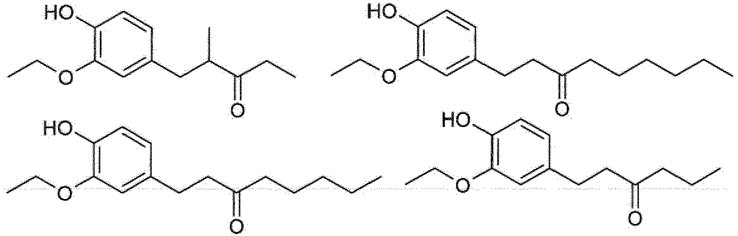

3. Zusammensetzung nach einem der vorhergehenden Ansprüche, wobei die Verbindung der Formel (I) aus den folgenden Verbindungen ausgewählt ist:

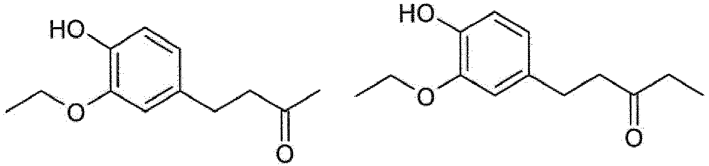

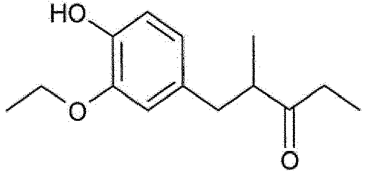

4. Zusammensetzung nach einem der vorhergehenden Ansprüche, wobei es sich bei der Verbindung der Formel (I) um die folgende Verbindung handelt:

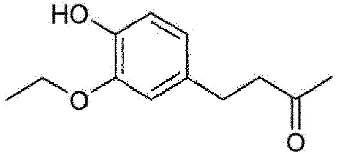

5. Zusammensetzung nach einem der vorhergehenden Ansprüche, wobei die Verbindung der Formel (I), allein oder als Mischung, in einem Anteil von 0,01 bis 5 Gew.-%, insbesondere 0,1 bis 2,5 Gew.-%, bezogen auf das Gewicht der Zusammensetzung, vorliegt.

6. Zusammensetzung nach einem der vorhergehenden Ansprüche, die in Form einer weißen oder farbigen Creme, einer Salbe, einer Milch, einer Lotion, eines Serums, einer Paste, eines Schaums oder eines Stifts vorliegt.

7. Zusammensetzung nach einem der vorhergehenden Ansprüche, umfassend ein physiologisch unbedenkliches Medium, das mindestens einen Bestandteil umfasst, der aus Silikon-Fettsubstanzen wie Silikonölen, -gummen und -wachsen; Nichtsilikon-Fettsubstanzen wie Ölen, Pasten und Wachsen pflanzlicher, mineralischer, tierischer und/oder synthetischer Herkunft; Fettsäuren mit 8 bis 32 Kohlenstoffatomen; synthetischen Estern und Ethern, insbesondere der Formel R¹COOR² und R¹OR², worin R¹ für den Rest einer Fettsäure mit 8 bis 29 Kohlenstoffatomen steht und R² für eine verzweigte oder unverzweigte Kohlenwasserstoffkette mit 3 bis 30 Kohlenstoffatomen steht; linearen und verzweigten Kohlenwasserstoffen mineralischer oder synthetischer Herkunft; Fettalkoholen mit 8 bis 26 Kohlenstoffatomen; Wasser; C2-C6-Alkoholen; Glykolen wie Propylenglykol, Ketonen; Verdickungsmitteln, Emulgatoren, Tensiden, Geliermitteln, kosmetischen Wirkstoffen, Duftstoffen, Füllstoffen, Farbstoffen, Feuchtigkeitsspendern, Vitaminen und Polymeren ausgewählt ist.

8. Zusammensetzung nach einem der vorhergehenden Ansprüche, die in Form eines Produkts zum Schminken der Haut des Gesichts, des Körpers oder der Lippen vorliegt.

9. Zusammensetzung nach einem der vorhergehenden Ansprüche, die in Form eines Shampoos vorliegt.

10. Zusammensetzung nach einem der vorhergehenden Ansprüche, die in Form einer Frisiercreme oder eines Frisiergels vorliegt.

11. Zusammensetzung nach einem der vorhergehenden Ansprüche, die in Form einer Färbezusammensetzung vorliegt.

12. Zusammensetzung nach einem der vorhergehenden Ansprüche, die in Form einer Lotion oder eines Gels gegen Haarausfall vorliegt.

## Claims

1. Cosmetic, dermatological or pharmaceutical composition comprising at least one compound of formula (I) : 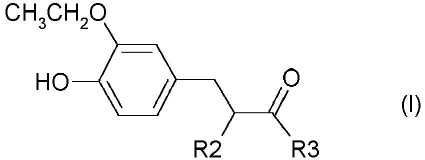 in which:
- either R2 represents a hydrogen atom and R3 represents a linear C1-C6 alkyl radical (saturated), optionally substituted with a hydroxyl group; or a linear C2-C6 alkenyl radical (C=C unsaturated), or alternatively a linear C2-C12 alkenyl radical substituted with a hydroxyl group;
- or R2 represents a methyl or ethyl radical and R3 represents a linear C1-C12 alkyl radical (saturated), optionally substituted with a hydroxyl group; or a linear C2-C12 alkenyl radical (C=C unsaturated), optionally substituted with a hydroxyl group;
the composition being in the form of aqueous-alcoholic solutions, oil-in-water or water-in-oil or multiple emulsions, aqueous gels, or dispersions of a fatty phase in an aqueous phase with the aid of spherules chosen from nanoparticles or lipid vesicles of ionic and/or nonionic type,
and
being in the form of a makeup product for facial skin, bodily skin or the lips; an aftershave gel or lotion; a hair-removing cream; a body hygiene composition such as a shower gel or a shampoo; a pharmaceutical composition; a solid composition such as a soap or a cleansing bar; an aerosol composition also comprising a pressurized propellant; a hairsetting lotion, a hairstyling cream or gel, a dye composition, a hair-restructuring lotion, a permanent-waving composition or a lotion or gel for preventing hair loss.

2. Composition according to the preceding claim, in which the compound of formula (I) is chosen from the following compounds:

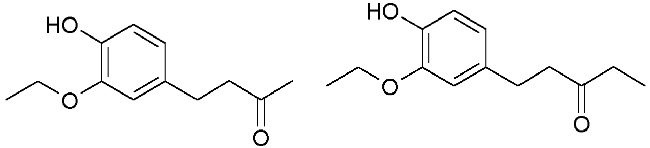

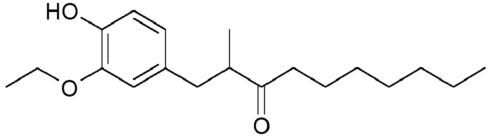

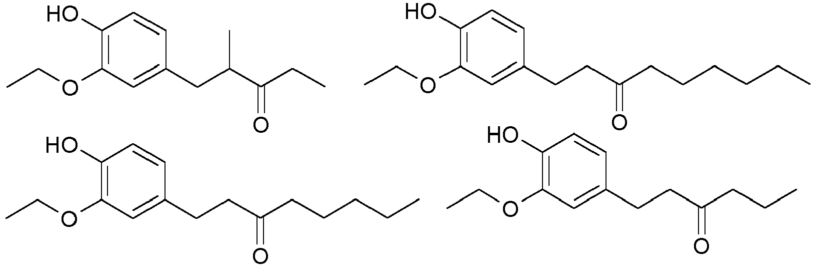

3. Composition according to either of the preceding claims, in which the compound of formula (I) is chosen from the following compounds:

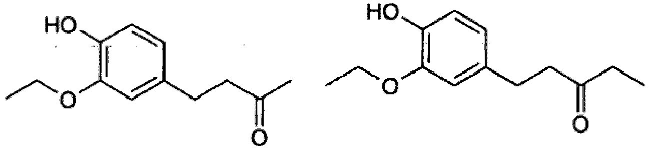

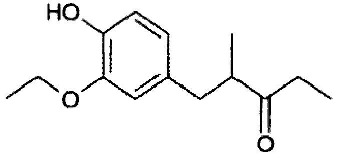

4. Composition according to one of the preceding claims, in which the compound of formula (I) is the compound:

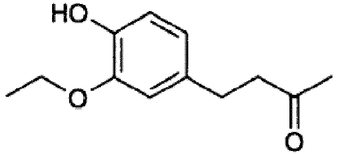

5. Composition according to one of the preceding claims, in which the compound of formula (I), alone or as a mixture, is present in a proportion of from 0.01% to 5% by weight and notably 0.1% to 2.5% by weight, relative to the weight of the composition.

6. Composition according to one of the preceding claims, which is in the form of a white or coloured cream, an ointment, a milk, a lotion, a serum, a paste, a foam or a stick.

7. Composition according to one of the preceding claims, comprising a physiologically acceptable medium which comprises at least one ingredient chosen from silicone fatty substances such as silicone oils, gums and waxes; non-silicone fatty substances such as oils, pastes and waxes of plant, mineral, animal and/or synthetic origin; fatty acids containing from 8 to 32 carbon atoms; synthetic esters and ethers, notably of formulae R¹COOR² and R¹OR² in which R¹ represents a fatty acid residue including from 8 to 29 carbon atoms, and R² represents a branched or unbranched hydrocarbon-based chain containing from 3 to 30 carbon atoms; linear or branched hydrocarbons of mineral or synthetic origin; fatty alcohols containing from 8 to 26 carbon atoms; water; C2-C6 alcohols; glycols such as propylene glycol, ketones; thickeners, emulsifiers, surfactants, gelling agents, cosmetic active agents, fragrances, fillers, dyestuffs, moisturizers, vitamins and polymers.

8. Composition according to one of the preceding claims, which is in the form of a makeup product for facial skin, bodily skin or the lips.

9. Composition according to one of the preceding claims, which is in the form of a shampoo.

10. Composition according to one of the preceding claims, which is in the form of a hairstyling cream or gel.

11. Composition according to one of the preceding claims, which is in the form of a dye composition.

12. Composition according to one of the preceding claims, which is in the form of a lotion or gel for preventing hair loss.
